# EUROPÄISCHE PATENTSCHRIFT

(11) **EP 0 603 130 B1**
(45) Veröffentlichungstag und Bekanntmachung des Hinweises auf die Patenterteilung: **05.09.2001**
(21) Anmeldenummer: 93810859.4
(22) Anmeldetag: 08.12.1993
(51) Int. Cl.: C07D 401/12, C07D 249/20, C07D 251/24, D06P 1/66, D06M 13/477, D06M 13/46

(54) **Kationische Verbindungen, deren Herstellung und deren Anwendung zur fotochemischen Stabilisierung basisch anfärbbarer Polyamidfasermaterialien**
Cationic compounds, their preparation and their use in the photochemical stabilisation of basic dyeable polyamide fibre materials
Composés cationiques, leur préparation et leur utilisation pour la stabilisation photochimique de matériaux de fibres polyamide, aptes à la teinture basique

(30) Priorität: 17.12.1992 CH 385092
(43) Veröffentlichungstag der Anmeldung: 22.06.1994
(73) Patentinhaber: Ciba Specialty Chemicals Holding Inc., 4057 Basel (CH)
(72) Erfinder: Adam, Jean-Marie, Dr., F-68300 Rosenau (FR)

(56) Entgegenhaltungen:
- EP-A- 0 357 545
- FR-A- 1 559 131
- EUR. POLYM. J., Bd.17, Nr.10, 1981 Seiten 1041 - 1048 J. ARCT ET AL
- CHEMICAL ABSTRACTS, vol. 115, no. 9, 1991, Columbus, Ohio, US; abstract no. 92278r, & PL-A-152 682 (POLITECHNIKA WROCLAWSKA)

## Beschreibung

Die vorliegende Erfindung betrifft neue kationische Verbindungen, deren Herstellung und deren Anwendung zur fotochemischen Stabilisierung basisch anfärbbarer Polyamidfasermaterialien sowie die zur Herstellung der Endprodukte benötigten neuen Ausgangsstoffe.

Bei "Differential-dyeing"-Teppichen stellt die mangelnde Lichtechtheit des basisch anfärbbaren Polyamidanteils, besonders die Vergrünung der Rotkomponente ein erhebliches Problem dar.

Es wurde nun gefunden, dass man das Problem weitgehend lösen kann, wenn man die Fasermaterialien mit Färbeflotten färbt, die zusätzlich neue kationische Verbindungen enthalten.

Die vorliegende Erfindung betrifft daher neue kationische Verbindungen der Formel 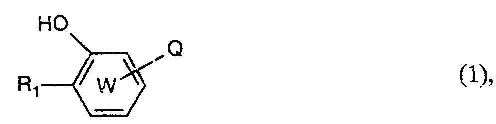 worin R₁ der Rest der Formel 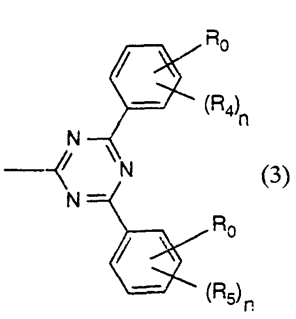 und
Q ein Rest der Formel 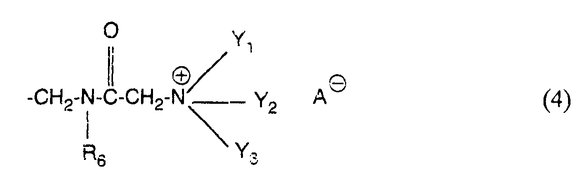 ist, wobei der Benzolring W ausser durch R₁, -OH und Q gegebenenfalls durch C₁-C₆-Alkyl und C₁-C₄-Alkoxy substituiert sein kann, und worin s
R₀ für Wasserstoff oder Hydroxy,
R₄ und R₅ unabhängig voneinander für Wasserstoff, C₁-C₄-Alkyl, C₁-C₄-Alkoxy oder Halogen, oder wenn R₀ Hydroxy und n 1 sind auch für die Gruppe der Formel Q,
R₆ für Wasserstoff oder C₁-C₄-Alkyl, und
n für die Zahl 1 oder 2 stehen,
Y₁ unsubstituiertes oder durch Halogen, Cyano, Hydroxy oder C₁-C₄-Alkoxy substituiertes C₁-C₄-Alkyl oder zusammen mit Y₂ und dem sie verbindenden N-Atom einen 5- bis 7-gliedrigen heterocyclischen Ring,
Y₂ unsubstituiertes oder durch Halogen, Cyano, Hydroxy oder C₁-C₄-Alkoxy substituiertes C₁-C₄-Alkyl oder zusammen mit Y₁ und dem sie verbindenden N-Atom einen 5- bis 7-gliedrigen heterocyclischen Ring,
Y₃ Wasserstoff, unsubstituiertes oder durch Cyano, Hydroxy, C₁-C₄-Alkoxy, Phenyl oder C₁-C₄-Alkoxycarbonyl substituiertes C₁-C₄-Alkyl oder C₃-C₄-Alkenyl,
oder Y₁, Y₂ und Y₃ zusammen mit dem sie verbindenden N-Atom einen gegebenenfalls durch C₁-C₄-Alkyl substituierten Pyridin- oder Chinolinrest, und
A^{⊖} ein farbloses Anion
bedeuten.

Stellt n die Zahl 1 und R₀ Hydroxy dar, dann ist der Hydroxyrest in 2-Stellung und die Gruppe Q in 4-Stellung gebunden. R₀ ist vorzugsweise Wasserstoff.

Als 5- bis 7-gliedrige heterocyclische Ringe, die Y₁ und Y₂ zusammen mit dem sie verbindenden N-Atom bilden können, kommt z.B. ein Morpholin-, Piperidin-, Pyrrolidin-und Hexamethyleniminring (= Hexahydro-1H-azepin) in Betracht.

Als 5- bis 7-gliedrige heterocyclische Ringe, die Y₁, Y₂ und Y₃ zusammen mit dem sie verbindenden N-Atom bilden können, kommt z.B. ein Pyridin-, Picolin-, insbesondere α-Picolin-, oder Chinolinring in Betracht.

Y₁, Y₂ und Y₃ sind vorzugsweise unsubstituiertes oder durch Hydroxy substituiertes C₁-C₄-Alkyl.

Von besonderem Interesse sind Verbindungen der Formel 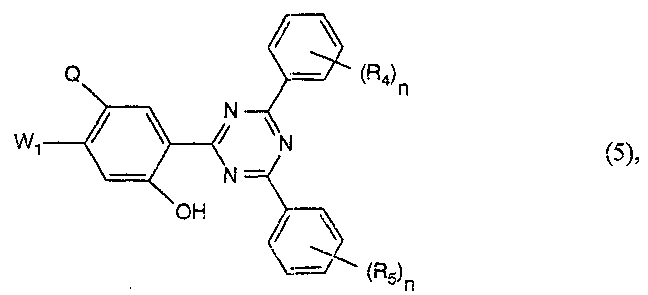 worin R₄ und R₅ Wasserstoff oder C₁-C₄-Alkyl bedeuten, Q und n die unter Formel (1) angegebenen Bedeutungen haben und W₁ C₁-C₄-Alkoxy, insbesondere Methoxy, ist.

Von ganz besonderem Interesse sind Verbindungen der Formel 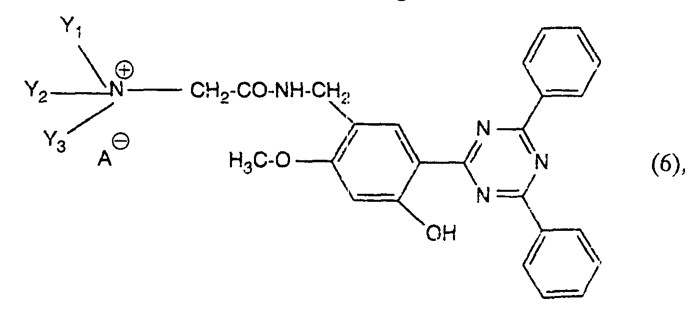 worin Y₁ und Y₂ unabhängig voneinander Methyl, Aethyl oder Hydroxyäthyl und Y₃ Wasserstoff, Methyl oder Aethyl ist, oder worin Y₁ und Y₂ zusammen mit dem sie verbindenden Stickstoffatom der Morpholinrest und Y₃ Wasserstoff oder Methyl ist, oder worin Y₁, Y₂ und Y₃ zusammen mit dem sie verbindenden Stickstoffatom Pyridinyl, durch Methyl substituiertes Pyridinyl, insbesondere α-Picolinyl, oder Chinolinyl ist, und A^{⊖} Cl^{⊖}, CH₃SO₄^{⊖} oder CH₃CO₂^{⊖} ist.

In den vorhergehenden Formeln nehmen die Substituenten folgende Bedeutungen an:

Halogen steht für Fluor, Brom und vorzugsweise Chlor.

C₁-C₄-Alkyl ist Methyl, Aethyl, n-Propyl, Isopropyl, n-Butyl, Isobutyl, sek. Butyl oder tert. Butyl.

C₁-C₄-Alkoxy bedeutet Methoxy, Aethoxy, n-Propoxy, Isopropoxy, n-Butoxy, Isobutoxy, sek. Butoxy oder tert. Butoxy.

C₂-C₉-Alkoxycarbonyl steht für Methoxycarbonyl, Aethoxycarbonyl, n-Propoxycarbonyl, n-Butoxycarbonyl, Pentoxycarbonyl, Hexyloxycarbonyl, Heptoxycarbonyl oder Octyloxycarbonyl.

Die neuen kationischen Verbindungen der Formel (1) werden dadurch hergestellt, dass man
a) zur Herstellung von Verbindungen der Formel (1), worin Y₁, Y₂ und Y₃ einen gegebenenfalls durch C₁-C₄-Alkyl substituierten Pyridin- oder Chinolinrest bedeuten, 1 Moläquivalent einer Verbindung der Formel 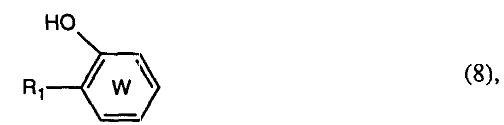 worin R₁ und W die unter Formel (1) angegebenen Bedeutungen haben, mit mindestens einem Moläquivalent einer den Rest der Formel

   -CH₂-N(R₆)-CO-CH₂-B (9)

   einführenden Verbindung, worin
   B eine Abgangsgruppe, wie z.B. Chlor, ist, und R₆ die unter Formel (4) angegebene Bedeutung hat, umsetzt, und anschliessend die gebildete Verbindung der Formel 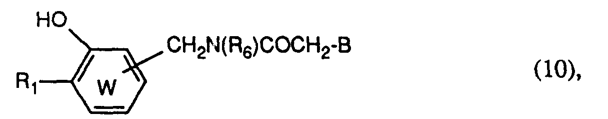 worin R₁, R₆, B und W die angegebenen Bedeutungen haben, mit einer den Rest der Formel 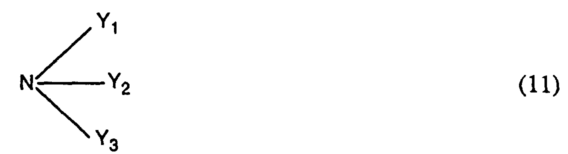 einführenden Verbindung, worin Y₁, Y₂ und Y₃ zusammen mit dem sie verbindenden N-Atom einen gegebenenfalls durch C₁-C₄-Alkyl substituierten Pyridin- oder Chinolinring bilden,umsetzt; oder
b) zur Herstellung von Verbindungen der Formel (1), worin Y₁, Y₂ und Y₃ eine der nicht unter die Verfahrensweise a) fallende Bedeutung hat, 1 Moläquivalent einer Verbindung der Formel 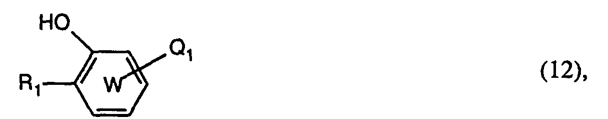 worin R₁ und W die unter Formel (1) angegebenen Bedeutungen haben, und Q₁ den Rest der Formel 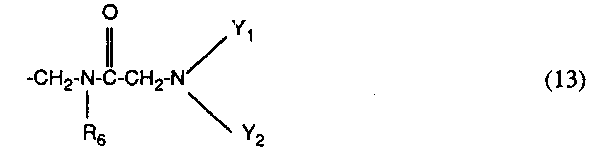 bedeutet, worin R₆, Y₁ und Y₂ die unter Formel (4) angegebenen Bedeutungen haben, mit mindestens einem Moläquivalent einer Verbindung der Formel

   Y₃-A (14),

   worin Y₃ und A die unter Formel (4) angegebenen Bedeutungen haben, bei einer Temperatur von 0 bis 180°C quaterniert bzw. protoniert.

Die Quaternierung bzw. Protonierung wird vorteilhaft bei Temperaturen zwischen 30 und 140°C durchgeführt.

Als Quaternierungs- bzw. Protonierungsmittel Y₃-A sind beispielsweise folgende Verbindungen geeignet: Alkylhalogenide wie Methyljodid, Aethyljodid, Aethylbromid, Butylbromid oder Benzylchlorid, Dialkylsulfate wie Dimethyl- oder Diäthylsulfat, Sulfonsäureester wie Toluol- oder Benzolsulfonsäuremethyl- oder -äthylester, Alkylenoxide wie Aethylen- oder Propylenoxide oder Epichlorhydrin, Acrylsäureester wie Acrylsäure-methyl-, -äthyl- oder -butylester, Acrylnitril, die Verbindungen der Formel 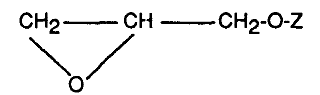 wobei Z für Methyl, Aethyl, Propyl, Butyl oder Phenyl steht. Phosphite oder Phosphonate der Formel 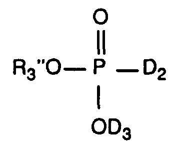 worin R₃" Alkyl mit 1 - 4 C-Atomen, D₂ Wasserstoff oder unsubstituiertes oder durch Hydroxy, Cyano, Alkylcarbonyloxy oder Alkoxycarbonyl mit jeweils 1 bis 4 C-Atomen im Alkylteil substituiertes Alkyl und D₃ Alkyl mit 1 bis 4 C-Atomen bedeuten.

Die Quaternierung der Verbindungen der Formel (12) mit Alkylhalogeniden, Dialkylsulfaten oder Sulfosäureestern zu den Verbindungen der Formel (1) erfolgt in Wasser oder vorzugsweise in einem gegenüber dem Alkylierungsmittel inerten Lösungsmittel. Geeignete Lösungsmittel sind z.B. Kohlenwasserstoffe wie Benzol, Toluol und Xylol, halogenierte aliphatische oder aromatische Kohlenwasserstoffe wie Chloroform, Aethylenchlorid, Chlorbenzol und Dichlorbenzol, Alkohole wie Aethanol, Butanol, Aethylenglykol und Aethylenglykolmonomethyläther, Aether wie Aethylenglykoldimethyläther und Dioxan oder Amide wie Dimethylformamid und N-Methylpyrrolidon.

Die Quaternierung mit den genannten Alkylierungsmitteln erfolgt vorteilhaft bei Temperaturen zwischen 0 und 180°C, vorzugsweise bei 30 bis 140°C.

Die Quaternierung der Verbindungen der Formel (12) zu den Verbindungen der Formel (1) mit Alkylenoxiden, Epichlorhydrin sowie dessen Derivaten der Formel 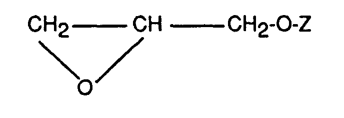 in der Z die genannten Bedeutungen hat, Acrylsäureester oder Acrylnitril wird bei den genannten Temperaturen in saurem Medium, vorteilhaft in Gegenwart einer organischen Säure wie Ameisensäure, Essigsäure, Propionsäure oder Benzoesäure durchgeführt, jedoch können auch anorganische Säuren wie Schwefelsäure, Phosphorsäure oder Halogenwasserstoffsäuren dafür verwendet werden. Diese anorganischen Säuren können in konzentrierter, handelsüblicher Form als verdünnte wässrige Lösungen oder in Mischung mit den genannten organischen Lösungsmitteln, gegebenenfalls unter Zusatz von Wasser, verwendet werden. Erfolgt die Umsetzung in Gegenwart von organischen Säuren, so wird meistens die konzentrierte Form dieser Säuren angewandt, gegebenenfalls in Mischung mit den genannten organischen Lösungsmitteln.

Bevorzugte Phosphite bzw. Phosphonate sind z.B. Dimethylphosphit, Diäthylphosphit, Dimethyl-methanphosphonat, Diäthylmethanphosphonat, Methyl-äthyl-methanphosphonat, Methyl-propyl-methan-phosphonat, Methyl-butyl-methanphosphonat, Methylhexyl-methanphosphonat, Methyl-octyl-methanphosphonat, Methyl-decyl-methanphosphonat, Methyl-dodecyl-methanphosphat, Dimethyl-β-hydroxy-äthanphosphonat, Dimethyl-β-acetoxyäthanphosphonat, Dimethyl-β-methoxycarbonyläthan-phosphonat und Dimethyl-β-cyanäthan-phosphonat.

Die Umsetzung erfolgt in Wasser und/oder organischen Lösungsmitteln wie Methanol, Aethanol, Propanol, Isopropanol, Butanol, Glykol, Glykolmethyläther, Glykol-di-methyläther, Glykolbutyläther, Diglykolmethyläther, Methyläthylketon, Methylbutylketon, Dimethylformamid, Sulfolan, Oxypropionitril, Toluol, Xylol, Benzylalkohol, Phenoxyäthanol, Benzyloxypropionitril bei vorzugsweise 60 bis 190°C. Bei der Verwendung von flüssigen Phosphiten oder Phosphonaten kann die Umsetzung auch in Abwesenheit eines zusätzlichen Lösungsmittels vorgenommen werden.

Werden protonierte Verbindungen der Formel (1) gewünscht, d.h. Säure-Additionssalze desselben, so verwendet man als Protonierungsmittel insbesondere Mineralsäuren. Geeignet sind prinzipiell alle starken bis mittelstarken organischen Säuren oder Mineralsäuren.

Als Lösungsmittel, in denen die Protonierung vorgenommen werden kann, eignen sich im allgemeinen alle inerten Lösungsmittel. Bevorzugt sind solche, die das Ausgangsprodukt lösen und aus denen sich das Endprodukt sofort ausscheidet. Beispielsweise seien genannt: Aromatische Kohlenwasserstoffe wie Benzol, Toluol und Xylol; Halogenkohlenwasserstoffe wie Trichloräthan, Tetrachloräthylen, Chlorbenzol oder Dichlorbenzol, ferner auch Nitroverbindungen wie Nitromethan, Nitropropan, Nitrobenzol, Alkanole und offene oder cyclische Aether wie Butanol, Dibutyläther, Aethylenglykol, Aethylenglykolmonomethyläther, Aethylenglykolmonoäthyläther, Anisol oder Dioxan; Ketone wie Cyclohexanon oder Methyläthylketon; Fettsäureamide wie Dimethylformamid oder Dimethylacetamid; Sulfoxyde wie Dimethylsulfoxyd und Carbonsäureester wie Essigester oder Essigsäurebutylester.

Die Verbindungen der Formel (8) sind bekannt und können in Analogie zu bekannten Verbindungen hergestellt werden.

Die den Rest der Formel (9) einführende Verbindungen sind ebenfalls bekannt und können in Analogie zu bekannten Verbindungen hergestellt werden. Als Beispiel für eine Verbindung der Formel (9) sei N-Hydroxymethyl-chloracetamid genannt.

Die Verbindungen der Formel (10) sind neu und stellen einen weiteren Gegenstand der vorliegenden Erfindung dar.

Die Verbindungen der Formel (11) sind bekannt. Als Beispiele seien genannt: Pyridin, Picolin, insbesondere α-Picolin, und Chinolin.

Die Verbindungen der Formel (12) sind ebenfalls neu und stellen daher einen weiteren Gegenstand der vorliegenden Erfindung dar.

Die Verbindungen der Formel (12) können z.B. dadurch hergestellt werden, dass man eine Verbindung der Formel 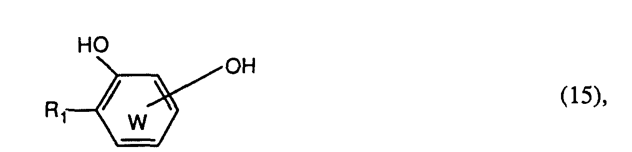 worin R₁ und W die angegebenen Bedeutungen haben mit einer Verbindung der Formel

Hal-CH₂-N(R₆)-CO-CH₂-N(Y₁)Y₂ (16),

worin Hal Chlor oder Brom bedeutet, und R₆, Y₁ und Y₂ die unter Formel (4) angegebene Bedeutung haben in Gegenwart einer Base, vorzugsweise Natrium- oder Kaliumhydroxyd umsetzt.

Die Verbindungen der Formeln (15) und (16) sind bekannt und können in Analogie zu bekannten Verbindungen hergestellt werden.

Die Umsetzung wird bei einer Temperatur zwischen 60 und 120°C, vorzugsweise 30 und 60°C vorgenommen.

Die Verbindungen der Formel (12) können aber auch dadurch hergestellt werden, dass man eine Verbindung der Formel 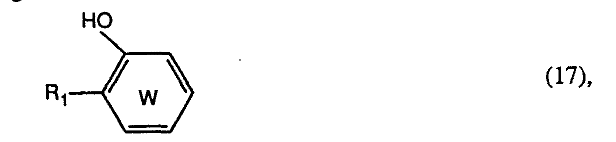 worin R₁ und W die angegebenen Bedeutungen haben,
a) mit einer Verbindung der Formel

   HO-CH₂-N(R₆)-CO-CH₂-B (18),

   worin B eine Abgangsgruppe, insbesondere Chlor oder Brom, ist und R₆ die unter Formel (4) angegebene Bedeutung hat, umsetzt, und
b) die erhaltene Verbindung der Formel 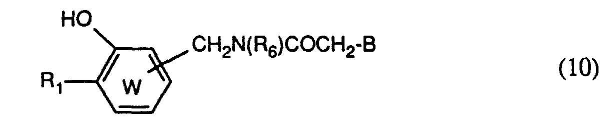 worin R₁, R₆, W und B die oben angegebenen Bedeutungen haben, mit einem sekundären oder tertiären Amin der Formel 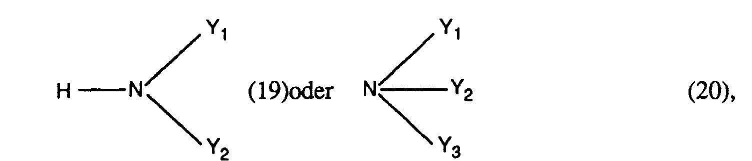 worin Y₁, Y₂ und Y₃ die unter Formel (4) angegebenen Bedeutungen haben, umsetzt.

Die Verbindungen der Formeln (17), (18), (19) und (20) sind bekannt und können in Analogie zu bekannten Verbindungen hergestellt werden.

Die erste Stufe a) wird bei einer Temperatur zwischen 60 und 120°C, und die zweite Stufe b) bei einer Temperatur zwischen 20 und 180°C vorgenommen.

Gegenstand der vorliegenden Erfindung sind demnach auch die Verfahren zur Herstellung von Verbindungen der Formeln (10) und (12).

Die erfindungsgemässen kationischen Lichtschutzmittel werden beim Färben von basisch anfärbbaren, d.h. sauer modifizierten Polyamidfasermaterialien eingesetzt, um die gefärbten Fasern gegen fotochemische Zersetzung zu stabilisieren und die Lichtechtheit der Färbungen zu erhöhen.

Die Fasermaterialien, die in Gegenwart des neuen Lichtschutzmittels gefärbt werden können, sind flächenförmig vor allem Bodenbeläge, wie z.B. Teppiche. Sie können ausser aus den oben erwähnten sauer modifizierten Fasermaterialien auch noch aus Gemischen von unmodifizierten (basischen) und sauer modifizierten Polyamidfasermaterialien bestehen. Diese Fasermaterialien sind auch als "Differential dyeing"- Polyamide bekannt und sind z.B. in W. Loy, Chemiefaserstoffe, Schiele und Schön, Berlin, 1978, Seiten 132-141 beschrieben.

Gegenstand der vorliegenden Erfindung ist demnach auch ein Verfahren zur Stabilisierung von basisch anfärbbaren Polyamidfasermaterialien. Das Verfahren ist dadurch gekennzeichnet, dass man dieses Fasermaterial mit einer Färbeflotte behandelt, die neben einem Dispersionsfarbstoff bzw. kationischen Farbstoff noch eine Verbindung der Formel (1) enthält.

Die für das erfindungsgemässe Verfahren geeigneten kationischen Farbstoffe können verschiedenen Farbstoffklassen angehören. Insbesondere handelt es sich um die gebräuchlichen Salze, beispielsweise Chloride, Sulfate oder Metallhalogenide, wie z.B. Zinkchloriddoppelsalze von kationischen Farbstoffen, deren kationischer Charakter z.B. von einer Carbonium-, Oxonium-, Sulfonium- oder vor allem Ammoniumgruppe herrührt. Beispiele für solche chromophore Systeme sind Azofarbstoffe, vor allem Monoazo- oder Hydrazonfarbstoffe, Diphenylmethan-, Triphenylmethan-, Methin- oder Azomethinfarbstoffe, Cumarin-, Ketonimin-, Cyanin-, Azin-, Xanthen-, Oxazin- oder Thiazinfarbstoffe. Schliesslich können auch Farbsalze der Anthrachinonreihe mit externer Oniumgruppe, beispielsweise einer Alkylammonium- oder Cycloammoniumgruppe sowie Benzo-1,2-pyranfarbsalze, die Cycloammoniumgruppen enthalten, verwendet werden.

Die zu verwendenden Dispersionsfarbstoffe, die in Wasser nur sehr wenig löslich sind und in der Farbflotte zum grössten Teil in Form einer feinen Dispersion vorliegen, können den verschiedensten Farbstoffklassen angehören, beispielsweise den Acridon-, Azo-, Anthrachinon-, Cumarin-, Methin-, Perinon-, Naphthochinonimin-, Chinophthalon-, Styryl- oder Nitrofarbstoffen.

Es können auch Mischungen von kationischen oder Dispersionsfarbstoffen erfindungsgemäss eingesetzt werden.

Ein weiterer Gegenstand der vorliegenden Erfindung ist ein Verfahren zur Stabilisierung von Mischungen aus sauer und basisch färbbaren Polyamidfasermaterialien. Das Verfahren ist dadurch gekennzeichnet, dass man dieses Fasermaterial mit einer Flotte behandelt, die neben einem kationischen Farbstoff und einer Verbindung der Formel (1) noch einen Säurefarbstoff enthält.

Bei den Säurefarbstoffen handelt es sich beispielsweise um Salze metallfreier Mono-, Dis-oder Polyazofarbstoffe einschliesslich der Formazanfarbstoffe sowie der Anthrachinon-, Xanthen-, Nitro-, Triphenylmethan- und Naphthochinoniminfarbstoffe. Der saure Charakter dieser Farbstoffe wird durch saure, salzbildende Substituenten, wie Carbonsäuregruppen, Schwefelsäure- und Phosphonsäureestergruppen, Phosphonsäuregruppen oder Sulfonsäuregruppen bedingt. Diese Farbstoffe können im Molekül auch sogenannte reaktive Gruppierungen, welche mit dem zu färbenden Material eine kovalente Bindung eingehen, aufweisen. Bevorzugt sind saure, eine einzige Sulfonsäuregruppe aufweisende Farbstoffe.

Es können auch Mischungen der Säurefarbstoffe eingesetzt werden. Beispielsweise können Farbstoffmischungen von mindestens 2 oder 3 Säurefarbstoffen verwendet werden.

Die Temperatur, bei der gefärbt wird, beträgt mindestens 70°C und in der Regel ist sie nicht höher als 106°C. Vorzugsweise liegt sie zwischen 80 und 130°C.

Die Menge der verwendeten Farbstoffe richtet sich nach der gewünschten Farbtiefe. Im allgemeinen haben sich Mengen von 0,001 bis 10 Gewichtsprozent, insbesondere 0,01 bis 5 Gewichtsprozent, bezogen auf das eingesetzte Fasermaterial, bewährt.

Als Fasermaterial sind synthetisches sauer modifiziertes Polyamid, allein oder auch in Mischungen zu erwähnen. Als synthetisches sauer modifiziertes Polyamid kommt z.B. solches aus Adipinsäure und Hexamethylendiamin (Polyamid 6,6), aus ∈-Caprolactam (Polyamid 6), aus ω-Aminoundecansäure (Polyamid 11), aus ω-Aminoönanthsäure (Polyamid 7), aus ω-Amino-pelargonsäure (Polyamid 8) oder aus Sebacinsäure und Hexamethylendiamin (Polyamid 6,10) in Betracht, das mit Carbonsäuren oder Sulfocarbonsäuren modifiziert worden ist.

Die zur Anwendung gelangenden Färbeflotten enthalten vorteilhafterweise Mineralsäuren, wie z.B. Schwefelsäure oder Phosphorsäure, oder organische Säuren, wie z.B. Ameisensäure, Essigsäure, Oxalsäure oder vorzugsweise Zitronensäure. Sie können auch Salze wie Ammoniumacetat, Ammoniumsulfat oder Natriumacetat enthalten. Die Säuren dienen vor allem zur Einstellung des pH-Wertes der Zubereitungen oder Flotten, der in der Regel 3 bis 7, vorzugsweise 3,5 bis 4,5 beträgt.

Zusätzlich zu den Lichtschutzmitteln, den Farbstoffen oder optischen Aufhellern können weitere in der Färbereitechnik übliche Hilfsmittel mitverwendet werden. Sie sind z.B. Dispergiermittel, Egalisiermittel, Elektrolyte, Netzmittel, Entschäumer, Schaumverhütungsmittel oder Verdicker.

Die zur Anwendung gelangenden Flotten können ausserdem noch photochemisch wirkende Antioxidantien wie Kupferkomplexe von Bisazomethinen enthalten. Diese Kupferkomplexe sind z.B. aus US-A-4,655,783 bekannt.

Die nachfolgenden Beispiele dienen zur Erläuterung der Erfindung. Darin sind die Teile Gewichtsteile und die Prozente Gewichtsprozente. Die Temperaturen sind in Celsiusgraden angegeben.

### Demonstration der Herstellung von Verbindungen der Formel (1) anhand analoger Umsetzungen:

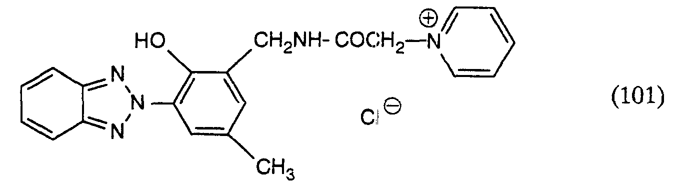

135 g der Verbindung der Formel 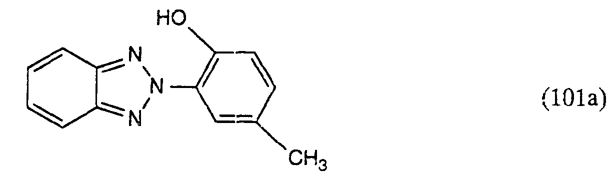 und 116 g N-Hydroxymethyl-chloracetamid werden homogenisiert und innert 90 Minuten bei 0 - 5° zu 660 ml Schwefelsäure (95 - 97%-ig) unter Rühren zugegeben. Es wird 2 Stunden bei 0 - 5° ausreagieren gelassen. Die viskose, gelbe Lösung wird dann innert 20 Minuten auf 3000 g einer Eis/Wasser-Mischung von 0 - 5° ausgetragen. Die erhaltene hellgelbe Suspension wird 30 Minuten verrührt, abfiltriert und anschliessend mit Wasser neutral gewaschen. Nach dem Trocknen erhält man 191 g (96,5% der Theorie) der Verbindung der Formel 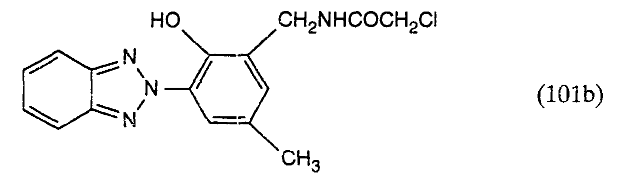 16,5 g der Verbindung der Formel (101b)
werden in 85 ml Pyridin zum Rückfluss erhitzt, wobei die dicke, weisse Suspension in Lösung geht. Nach 3 Minuten fällt das Produkt kristallin aus. Anschliessend wird die Suspension 10 Minuten verrührt, abfiltriert und mit Toluol gewaschen. Der Rückstand wird bei 90° getrocknet. Es werden 19,0 g (93% der Theorie) der Verbindung der Formel (101) erhalten.

Wenn man wie oben angegeben verfährt, jedoch anstelle von Pyridin eine äquimolare Menge Methylpyridin, Trimethylamin, Diethylmethylamin, Dimethyl-β-hydroxyethylamin, Morpholin, Diethylamin, Dimethylamin oder Chinolin, und als Verbindung der Formel (101a) eine äquimolare Menge der Verbindung der Formel 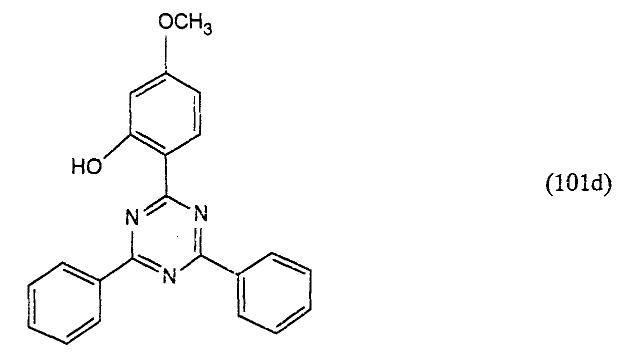 einsetzt, erhält man die in der nachfolgenden Tabelle angegebenen Verbindungen, wobei die Verbindungen auch dadurch hergestellt werden, dass man die Verbindung der Formel (101b) mit einem entsprechenden Amin umsetzt, und anschliessend quaterniert oder protoniert, wie oben angegeben.

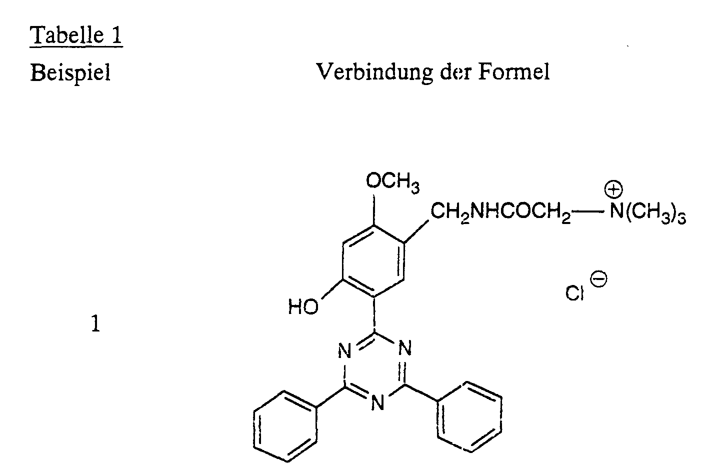

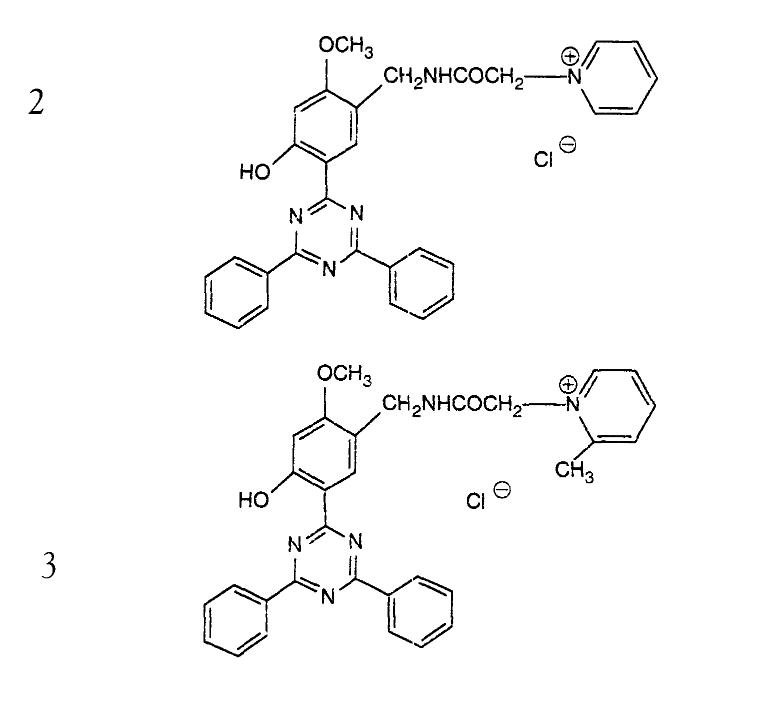

## Patentansprüche

1. Kationische Verbindungen der Formel 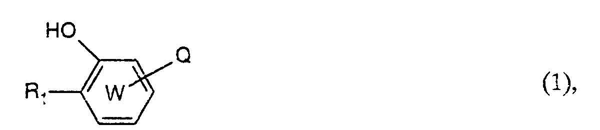 worin R₁ der Rest der Formel 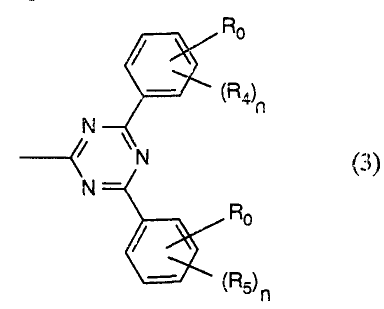 und
Q ein Rest der Formel 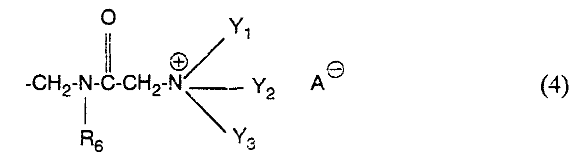 ist, wobei der Benzolring W ausser durch R₁, -OH und Q gegebenenfalls durch C₁-C₆-Alkyl und C₁-C₄-Alkoxy substituiert sein kann, und worin
R₀ für Wasserstoff oder Hydroxy,
R₄ und R₅ unabhängig voneinander für Wasserstoff, C₁-C₄-Alkyl, C₁-C₄-Alkoxy oder Halogen, oder wenn R₀ Hydroxy und n 1 sind auch für die Gruppe der Formel Q,
R₆ für Wasserstoff oder C₁-C₄-Alkyl, und
n für die Zahl 1 oder 2 stehen,
Y₁ unsubstituiertes oder durch Halogen, Cyano, Hydroxy oder C₁-C₄-Alkoxy substituiertes C₁-C₄-Alkyl oder zusammen mit Y₂ und dem sie verbindenden N-Atom einen 5- bis 7-gliedrigen heterocyclischen Ring,
Y₂ unsubstituiertes oder durch Halogen, Cyano, Hydroxy oder C₁-C₄-Alkoxy substituiertes C₁-C₄-Alkyl oder zusammen mit Y₁ und dem sie verbindenden N-Atom einen 5- bis 7-gliedrigen heterocyclischen Ring,
Y₃ Wasserstoff, unsubstituiertes oder durch Cyano, Hydroxy, C₁-C₄-Alkoxy, Phenyl oder C₁-C₄-Alkoxycarbonyl substituiertes C₁-C₄-Alkyl oder C₃-C₄-Alkenyl,
oder Y₁, Y₂ und Y₃ zusammen mit dem sie verbindenden N-Atom einen gegebenenfalls durch C₁-C₄-Alkyl substituierten Pyridin- oder Chinolinrest, und
A^{⊖} ein farbloses Anion
bedeuten.

2. Verbindungen gemäss Anspruch 1, worin Y₁und Y₂ zusammen mit dem sie verbindenden N-Atom einen Morpholin-, Pyrrolidin-, Piperidin- oder Hexamethyleniminring als 5- bis 7-gliedrigen heterocyclischen Ring bedeuten.

3. Verbindungen gemäss Anspruch 1, worin Y₁, Y₂ und Y₃ zusammen mit dem sie verbindenden N-Atom einen Pyridin-, Picolin- oder Chinolinring bedeuten.

4. Verbindungen gemäss Anspruch 1 der Formel 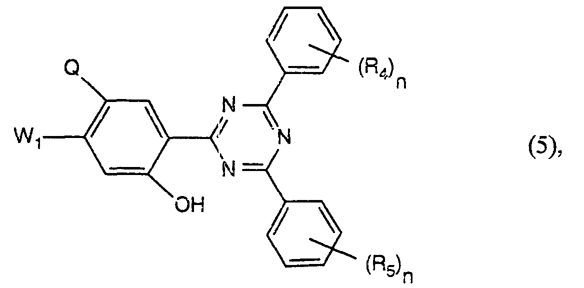 worin R₄ und R₅ Wasserstoff oder C₁-C₄-Alkyl bedeuten, Q und n die in Anspruch 1 angegebenen Bedeutungen haben, und W₁ C₁-C₄-Alkoxy, insbesondere Methoxy, ist.

5. Verbindungen gemäss Anspruch 4 der Formel 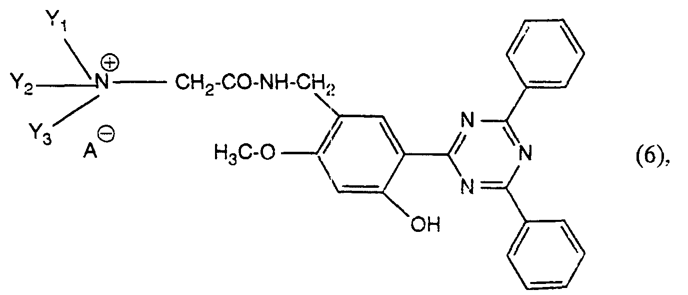 worin Y₁ und Y₂ unabhängig voneinander Methyl, Aethyl oder Hydroxyäthyl und Y₃ Wasserstoff, Methyl oder Aethyl ist, oder worin Y₁ und Y₂ zusammen mit dem sie verbindenden Stickstoffatom den Morpholinrest und Y₃ Wasserstoff oder Methyl ist, oder worin Y₁, Y₂ und Y₃ zusammen mit dem sie verbindenden Stickstoffatom Pyridinyl, durch Methyl substituiertes Pyridinyl oder Chinolinyl ist, und A^{⊖} ein Cl^{⊖-}, CH₃SO₄^{⊖-} oder CH₃CO₂^{⊖}-Anion ist.

6. Verfahren zur Herstellung der kationischen Verbindungen gemäss Anspruch 1, **dadurch gekennzeichnet, dass** man eine Verbindung der Formel 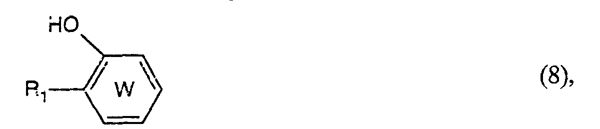 worin R₁ und W die in Anspruch 1 angegebenen Bedeutungen haben, mit mindestens einem Moläquivalent einer den Rest der Formel
-CH₂-N(R₆)-CO-CH₂-B (9)
einführenden Verbindung, worin B eine Abgangsgruppe, insbesondere Chlor ist, und R₆ die in Anspruch 1 angegebene Bedeutung hat, umsetzt, und anschliessend die gebildete Verbindung der Formel 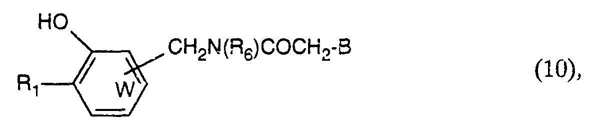 worin R₁, R₆, B und W die angegebenen Bedeutungen haben, mit einer den Rest der Formel 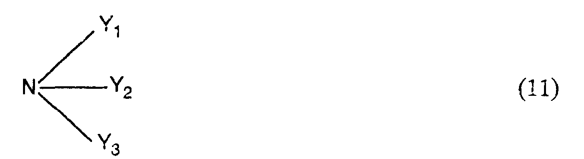 einführenden Verbindung, worin Y₁, Y₂ und Y₃ zusammen mit dem sie verbindenden Stickstoffatom einen gegebenenfalls durch C₁-C₄-Alkyl substituierten Pyridin- oder Chinolinring bedeuten, umsetzt.

7. Verfahren zur fotochemischen Stabilisierung von basisch anfärbbaren Polyamidfasermaterialien mit Lichtschutzmitteln, **dadurch gekennzeichnet, dass** man das basisch anfärbbare Fasermaterial mit einer Färbeflotte behandelt, die neben einem Dispersionsfarbstoff bzw. kationischen Farbstoff eine Verbindung der Formel 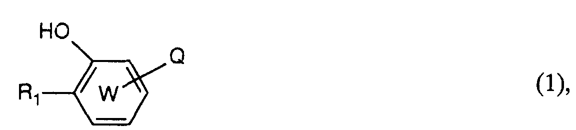 worin R₁ der Rest der Formel 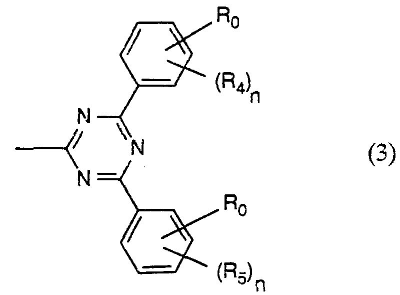 und
Q ein Rest der Formel 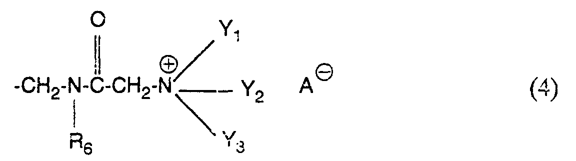 ist, wobei der Benzolring W ausser durch R₁, -OH und Q gegebenenfalls durch C₁-C₆-Alkyl und C₁-C₄-Alkoxy substituiert sein kann, und worin
R₀ für Wasserstoff oder Hydroxy,
R₄ und R₅ unabhängig voneinander für Wasserstoff, C₁-C₄-Alkyl, C₁-C₄-Alkoxy oder Halogen, oder wenn R₀ Hydroxy und n 1 sind auch für die Gruppe der Formel Q,
R₆ für Wasserstoff oder C₁-C₄-Alkyl, und
n für die Zahl 1 oder 2 stehen,
Y₁ unsubstituiertes oder durch Halogen, Cyano, Hydroxy oder C₁-C₄-Alkoxy substituiertes C₁-C₄-Alkyl oder zusammen mit Y₂ und dem sie verbindenden N-Atom einen 5- bis 7-gliedrigen heterocyclischen Ring,
Y₂ unsubstituiertes oder durch Halogen, Cyano, Hydroxy oder C₁-C₄-Alkoxy substituiertes C₁-C₄-Alkyl oder zusammen mit Y₁ und dem sie verbindenden N-Atom einen 5- bis 7-gliedrigen heterocyclischen Ring,
Y₃ Wasserstoff, unsubstituiertes oder durch Cyano, Hydroxy, C₁-C₄-Alkoxy, Phenyl oder C₁-C₄-Alkoxycarbonyl substituiertes C₁-C₄-Alkyl oder C₃-C₄-Alkenyl,
oder Y₁, Y₂ und Y₃ zusammen mit dem sie verbindenden N-Atom den gegebenenfalls durch C₁-C₄-Alkyl substituierten Pyridin- oder Chinolinring und
A^{⊖} ein farbloses Anion
bedeuten, enthält.

8. Verfahren gemäss Anspruch 7 zur Stabilisierung von basisch anfärbbaren Polyamidfasermaterialien, **dadurch gekennzeichnet, dass** die Färbeflotte noch ein Kupferkomplex eines Bisazomethins enthält.

9. Verfahren zur fotochemischen Stabilisierung von mit kationischen Farbstoffen anfärbbaren Polyacrylnitrilfasermaterialien, **dadurch gekennzeichnet, dass** man diese Materialien mit Flotten färbt, welche neben einem kationischen Farbstoff noch eine Verbindung der in Anspruch 1 definierten Formel (1) enthält.

10. Verwendung der in Anspruch 1 definierten kationischen Verbindungen zur fotochemischen Stabilisierung von basisch anfärbbaren Polyamidfasermaterialien.

11. Das gemäss dem Verfahren nach einem der Ansprüche 7 bis 10 stabilisierte basisch anfärbbare Polyamidfasermaterial.

## Claims

1. A cationic compound of the formula 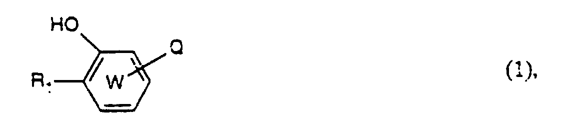 in which R₁ is the radical of the formula 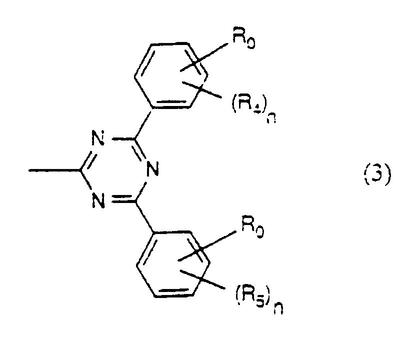 and
Q is a radical of the formula 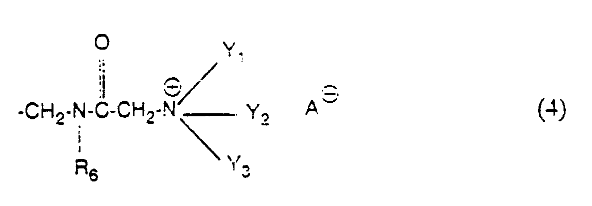 where the benzene ring W, in addition to being substituted by R₁, -OH and Q, may be unsubstituted or substituted by C₁-C₆alkyl and C₁-C₄alkoxy, and in which
R₀ is hydrogen or hydroxyl,
R₄ and R₅ are each independently of the other hydrogen, C₁-C₄alkyl, C₁-C₄alkoxy or halogen, or, if R₀ is hydroxyl and n is 1, are alternatively the group of the formula Q,
R₆ is hydrogen or C₁-C₄alkyl, and
n is the number 1 or 2,
Y₁ is unsubstituted C₁-C₄alkyl or C₁-C₄alkyl which is substituted by halogen, cyano, hydroxyl or C₁-C₄alkoxy, or together with Y₂ and the N atom linking them is a 5-to 7-membered heterocyclic ring,
Y₂ is unsubstituted C₁-C₄alkyl or C₁-C₄alkyl which is substituted by halogen, cyano, hydroxyl or C₁-C₄alkoxy, or together with Y₁ and the N atom linking them is a 5-to 7-membered heterocyclic ring,
Y₃ is hydrogen, unsubstituted C₁-C₄alkyl or C₃-C₄alkenyl, or C₁-C₄alkyl or C₃-C₄alkenyl which is substituted by cyano, hydroxyl, C₁-C₄alkoxy, phenyl or C₁-C₄alkoxy-carbonyl,
or Y₁, Y₂ and Y₃, together with the N atom linking them, are an unsubstituted or C₁-C₄alkyl-substituted pyridine or quinoline radical, and
A^{⊖} is a colourless anion.

2. A compound according to claim 1, in which Y₁ and Y₂, together with the N atom linking them, are a morpholine, pyrrolidine, piperidine or hexamethyleneimine ring as a 5- to 7--membered heterocyclic ring.

3. A compound according to claim 1, in which Y₁, Y₂ and Y₃, together with the N atom linking them, are a pyridine, picoline or quinoline ring.

4. A compound according to claim 1 of the formula 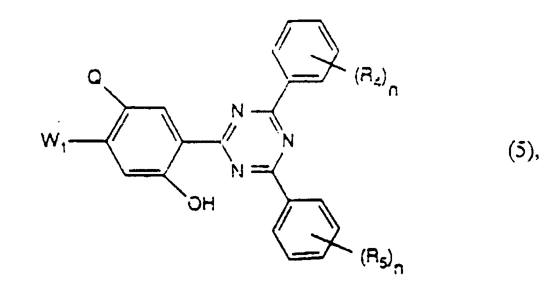 in which R₄ and R₅ are hydrogen or C₁-C₄alkyl, Q and n are as defined in claim 1, and W₁ is C₁-C₄alkoxy, especially methoxy.

5. A compound according to claim 4 of the formula 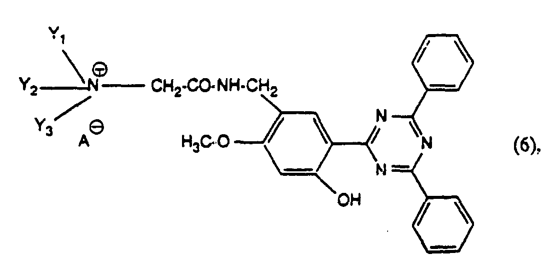 in which Y₁ and Y₂ independently of one another are methyl, ethyl or hydroxyethyl and Y₃ is hydrogen, methyl or ethyl, or in which Y₁ and Y₂, together with the nitrogen atom linking them, are the morpholine radical and Y₃ is hydrogen or methyl, or in which Y₁, Y₂ and Y₃, together with the nitrogen atom linking them, are pyridinyl, methyl-substituted pyridinyl or quinolinyl, and A^{⊖} is a Cl^{⊖}, CH₃SO₄^{⊖} or CH₃CO₂^{⊖} anion.

6. A process for preparing a cationic compound according to claim 1, which comprises reacting a compound of the formula 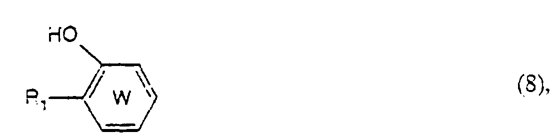 in which R₁ and W are as defined in claim 1, with at least one molar equivalent of a compound that introduces the radical of the formula
-CH₂-N(R₆)-CO-CH₂-B (9)
in which B is a leaving group, especially chloro, and R₆ is as defined in claim 1, and subsequently reacting the resulting compound of the formula 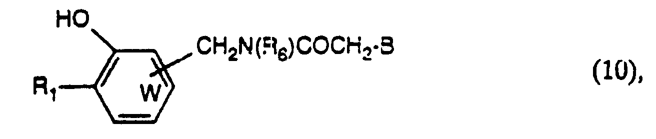 in which R₁, R₆, B and W are as defined, with a compound that introduces the radical of the formula 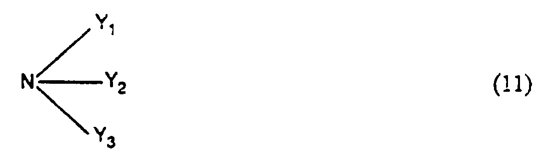 in which Y₁, Y₂ and Y₃, together with the nitrogen atom linking them, are an unsubstituted or C₁-C₄alkyl-substituted pyridine or quinoline ring.

7. A process for the photochemical stabilization of basic dyeable polyamide fibre materials with light stabilizers, which comprises treating the basic dyeable fibre material with a dyeing liquor comprising a disperse dye and/or cationic dye and also a compound of the formula 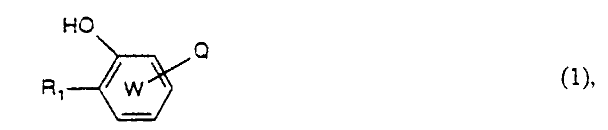 in which R₁ is the radical of the formula 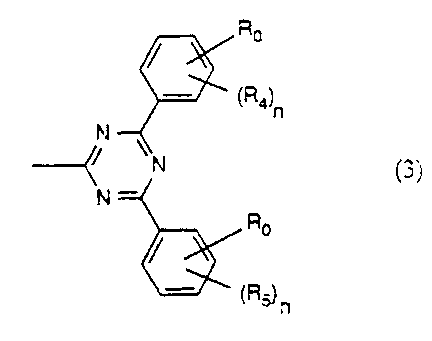 and
Q is a radical of the formula 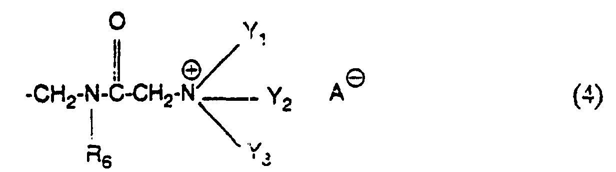 where the benzene ring W, in addition to being substituted by R₁, -OH and Q, may be unsubstituted or substituted by C₁-C₆alkyl and C₁-C₄alkoxy, and in which
R₀ is hydrogen or hydroxyl,
R₄ and R₅ are each independently of the other hydrogen, C₁-C₄alkyl, C₁-C₄alkoxy or halogen, or, if R₀ is hydroxyl and n is 1, are alternatively the group of the formula Q,
R₆ is hydrogen or C₁-C₄alkyl, and
n is the number 1 or 2,
Y₁ is unsubstituted C₁-C₄alkyl or C₁-C₄alkyl which is substituted by halogen, cyano, hydroxyl or C₁-C₄alkoxy, or together with Y₂ and the N atom linking them is a 5-to 7-membered heterocyclic ring,
Y₂ is unsubstituted C₁-C₄alkyl or C₁-C₄alkyl which is substituted by halogen, cyano, hydroxyl or C₁-C₄alkoxy, or together with Y₁ and the N atom linking them is a 5-to 7-membered heterocyclic ring,
Y₃ is hydrogen, unsubstituted C₁-C₄alkyl or C₃-C₄alkenyl, or C₁-C₄alkyl or C₃-C₄alkenyl which is substituted by cyano, hydroxyl, C₁-C₄alkoxy, phenyl or C₁-C₄alkoxy-carbonyl,
or Y₁, Y₂ and Y₃, together with the N atom linking them, are an unsubstituted or C₁-C₄alkyl-substituted pyridine or quinoline radical, and
A^{⊖} is a colourless anion.

8. A process according to claim 7 for stabilizing basic dyeable polyamide fibre materials, wherein the dyeing liquor further comprises a copper complex of a bisazomethine.

9. A process for the photochemical stabilization of polyacrylonitrile fibre materials dyeable with cationic dyes, which comprises dyeing said materials with liquors comprising a cationic dye and also a compound of the formula (1) defined in claim 1.

10. The use of a cationic compound defined in claim 1 for the photochemical stabilization of basic dyeable polyamide fibre materials.

11. Basic dyeable polyamide fibre material stabilized by the process as claimed in any one of claims 7 to 10.

## Revendications

1. Composés cationiques de formule 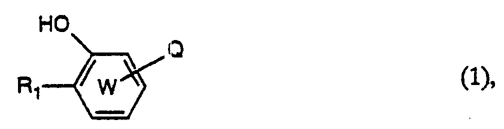 dans laquelle R₁ représente le groupe de formule 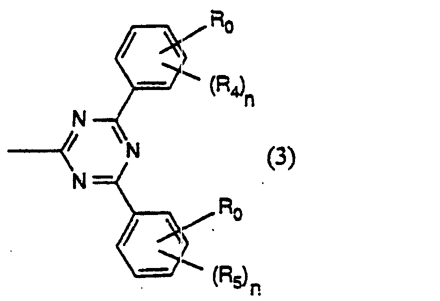 et
Q un groupe de formule 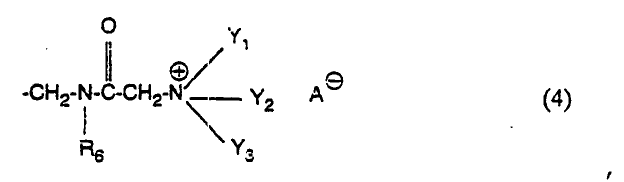 dans laquelle le noyau benzénique W en plus de R₁, -OH et
Q peut éventuellement être substitué par un groupe alkyle en C₁ à C₆ et alcoxy en C₁ à C₄, et dans laquelle
R₀ représente un atome d'hydrogène ou un groupe hydroxy,
R₄ et R₅ représentent indépendamment l'un de l'autre un atome d'hydrogène, un groupe alkyle en C₁ à C₄, alcoxy en C₁ à C₄ ou un atome d'halogène, ou lorsque R₀ est hydroxy et n est 1 peuvent aussi représenter un groupe de formule Q,
R₆ représente un atome d'hydrogène ou un groupe alkyle en C₁ à C₄, et
n représente le nombre 1 ou 2,
Y₁ représente un groupe alkyle en C₁ à C₄ non substitué ou substitué par un atome d'halogène, un groupe cyano, hydroxy ou alcoxy en C₁ à C₄ ou ensemble avec Y₂ et l'atome d'azote les liant un hétérocycle à 5 à 7 chaînons,
Y₂ représente un groupe alkyle en C₁ à C₄ non substitué ou substitué par un atome d'halogène, un groupe cyano, hydroxy ou alcoxy en C₁ à C₄ ou ensemble avec Y₁ et l'atome d'azote les liant un hétérocycle à 5 à 7 chaînons,
Y₃ représente un atome d'hydrogène, un groupe alkyle en C₁ à C₄ ou alcényle en C₃ à C₄ non substitué ou substitué par un groupe cyano, hydroxy ou alcoxy en C₁ à C₄, phényle ou alcoxy en C₁ à C₄-carbonyle,
ou Y₁, Y₂ et Y₃ représentent ensemble avec l'atome d'azote les liant un groupe pyridine ou quinoléine éventuellement substitué par un groupe alkyle en C₁ à C₄, et
A^{⊖} un anion incolore.

2. Composés selon la revendication 1, dans lesquels Y₁ et Y₂ représentent ensemble avec l'atome d'azote les liant un cycle morpholine, pyrrolidine, pipéridine ou hexaméthylènimine comme hétérocycle à 5 à 7 chaînons.

3. Composés selon la revendication 1, dans lesquels Y₁, Y₂ et Y₃ représentent ensemble avec l'atome d'azote les liant un cycle pyridine, picoline ou quinoléine.

4. Composés selon la revendication 1 de formule 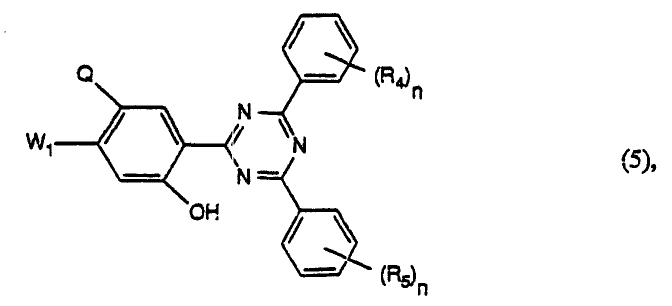 dans laquelle R₄ et R₅ représentent un atome d'hydrogène ou un groupe alkyle en C₁ à C₄, Q et n ont les significations indiquées à la revendication 1 et W₁ représente un groupe alcoxy en C₁ à C₄, en particulier méthoxy.

5. Composés selon la revendication 4 de formule 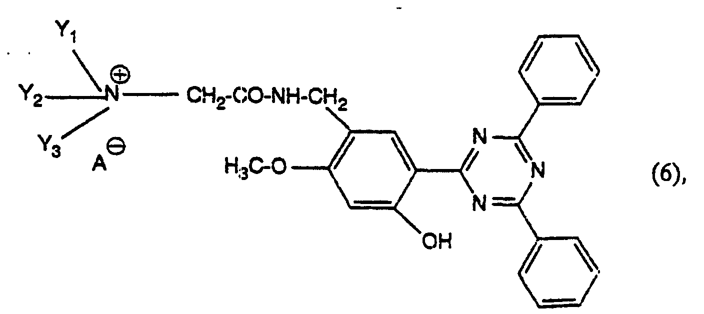 dans laquelle Y₁ et Y₂ représentent indépendamment l'un de l'autre un groupe méthyle, éthyle ou hydroxyéthyle et Y₃ est un atome d'hydrogène, un groupe méthyle ou éthyle, ou dans laquelle Y₁ et Y₂ sont ensemble avec l'atome d'azote les liant le groupe morpholine et Y₃ est un atome d'hydrogène ou un groupe méthyle, ou dans laquelle Y₁, Y₂ et Y₃ sont ensemble avec l'atome d'azote les liant le groupe pyridinyle, pyridinyle substitué par des groupes méthyle ou quinoléinyle, et A^{⊖} est un anion Cl^{⊖}, CH₃SO₄^{⊖} ou CH₃CO₂^{⊖}.

6. Procédé pour la préparation de composés cationiques selon la revendication 1, **caractérisé en ce qu'**on met à réagir un composé de formule 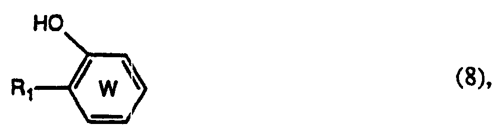 dans laquelle R₁ et W ont les significations indiquées à la revendication 1, avec au moins un équivalent molaire d'un composé introduisant le groupe de formule
**-CH**_{**2**}**-N(R**_{**6**}**)-CO-CH**_{**2**}**-B** (9),
dans laquelle B est un groupe partant, en particulier le chlore, et R₆ a la signification indiquée à la revendication 1, et en ce qu'on met à réagir ensuite le composé formé de formule 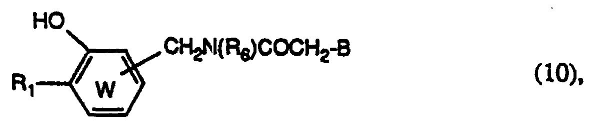 dans laquelle R₁, R₆, B et W ont les significations indiquées, avec un composé introduisant le groupe de formule 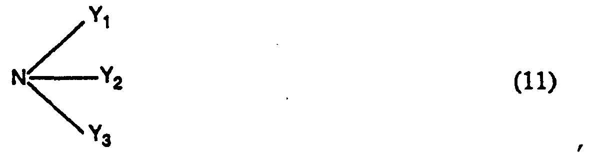 dans laquelle Y₁, Y₂ et Y₃ forment ensemble avec l'atome d'azote les liant un cycle pyridine ou quinoléine éventuellement substitué par un groupe alkyle en C₁ à C₄.

7. Procédé pour la stabilisation photochimique de matériaux en fibres polyamide aptes à la coloration basique avec des agents de protection contre la lumière,
**caractérisé en ce qu'**on traite le matériau en fibres apte à la coloration basique avec un bain de teinture, qui contient en plus d'un colorant de dispersion ou cationique un composé de formule 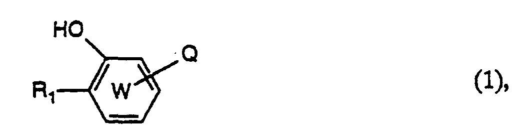 dans laquelle R₁ représente le groupe de formule 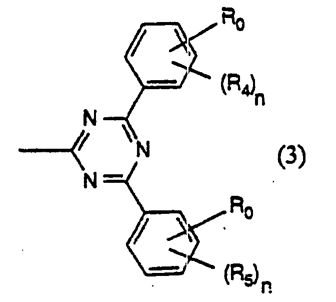 et
Q un qroupe de formule 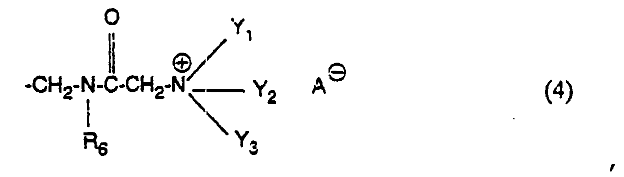 dans laquelle le noyau benzénique W en plus de R₁, -OH et Q peut éventuellement être substitué par un groupe alkyle en C₁ à C₆ et alcoxy en C₁ à C₄, et dans laquelle
R₀ représente un atome d'hydrogène ou un groupe hydroxy,
R₄ et R₅ représentent indépendamment l'un de l'autre un atome d'hydrogène, un groupe alkyle en C₁ à C₄, alcoxy en C₁ à C₄ ou un atome d'halogène, ou lorsque R₀ est hydroxy et n est 1 peuvent aussi représenter un groupe de formule Q,
R₆ représente un atome d'hydrogène ou un groupe alkyle en C₁ à C₄, et
n représente le nombre 1 ou 2,
Y₁ représente un groupe alkyle en C₁ à C₄ non substitué ou substitué par un atome d'halogène, un groupe cyano, hydroxy ou alcoxy en C₁ à C₄ ou ensemble avec Y₂ et l'atome d'azote les liant un hétérocycle à 5 à 7 chaînons,
Y₂ représente un groupe alkyle en C₁ à C₄ non substitué ou substitué par un atome d'halogène, un groupe cyano, hydroxy ou alcoxy en C₁ à C₄ ou ensemble avec Y₁ et l'atome d'azote les liant un hétérocycle à 5 à 7 chaînons,
Y₃ représente un atome d'hydrogène, un groupe alkyle en C₁ à C₄ ou alcényle en C₃ à C₄ non substitué ou substitué par un groupe cyano, hydroxy ou alcoxy en C₁ à C₄, phényle ou alcoxy en C₁ à C₄-carbonyle,
ou Y₁, Y₂ et Y₃ représentent ensemble avec l'atome d'azote les liant un groupe pyridine ou quinoléine éventuellement substitué par un groupe alkyle en C₁ à C₄, et
A^{⊖} un anion incolore.

8. Procédé selon la revendication 7 pour la stabilisation de matériaux en fibres polyamide aptes à la coloration basique, **caractérisé en ce que** le bain de teinture contient encore un complexe de cuivre d'une bis-azométhine.

9. Procédé pour la stabilisation photochimique de matériaux en fibre de polyacrylonitrile aptes à la coloration par des colorants cationiques, **caractérisé en ce qu'**on teinte ces matériaux avec des bains, qui contiennent en plus d'un colorant cationique encore un composé de la formule (1) définie à la revendication 1.

10. Utilisation des composés cationiques définis à la revendication 1 pour la stabilisation photochimique de matériaux en fibre polyamide aptes à la coloration basique.

11. Matériau en fibres de polyamide apte à la coloration basique stabilisé avec le procédé selon l'une des revendications 7 à 10.
